# EUROPEAN PATENT SPECIFICATION

(11) **EP 2 770 317 B1**
(45) Date of publication and mention of the grant of the patent: **20.09.2017**
(21) Application number: 13156227.4
(22) Date of filing: 21.02.2013
(51) Int. Cl.: G01N 15/05, G01F 23/292, G01N 15/04

(54) **APPARATUS FOR DETERMINING A VERTICAL POSITION OF AT LEAST ONE INTERFACE BETWEEN A FIRST COMPONENT AND AT LEAST ONE SECOND COMPONENT AND LABORATORY AUTOMATION SYSTEM**
VORRICHTUNG ZUM BESTIMMEN EINER VERTIKALEN POSITION VON MINDESTENS EINER ZWISCHENSCHICHT ZWISCHEN EINER ERSTEN KOMPONENTE UND MINDESTENS EINER ZWEITEN KOMPONENTE UND LABORAUTOMATISIERUNGSSYSTEM
APPAREIL PERMETTANT DE DÉTERMINER UNE POSITION VERTICALE D'AU MOINS UNE INTERFACE ENTRE UN PREMIER COMPOSANT ET AU MOINS UN DEUXIÈME COMPOSANT ET SYSTÈME D'AUTOMATISATION DE LABORATOIRE

(43) Date of publication of application: 27.08.2014
(73) Proprietor: Roche Diagnostics GmbH, 68305 Mannheim (DE); F. Hoffmann-La Roche AG, 4070 Basel (CH)
(72) Inventor: Köhler, Michael, 71364 Winnenden (DE); Klinec, Darko, 75365 Calw (DE)
(74) Representative: Patentanwälte Ruff, Wilhelm, Beier, Dauster & Partner mbB

(56) References cited:
- WO-A1-2011/019576
- US-A1- 2003 141 456
- US-A1- 2004 260 520
- US-A1- 2005 205 788

## Description

The invention relates to a laboratory automation system comprising an improved apparatus for determining a vertical position of at least one interface between a first component and at least one second component, the components being comprised as different layers in a sample container.

US 2004/0260520 A1 discloses an apparatus for determining a vertical position of at least one interface between a first component and at least one second component, the components being comprised as different layers in a sample container or sample tube. The measurement is based on a single optical channel which guides a light wave comprising a first and a second optical wavelength emitted by corresponding laser diodes.

US 2005/0205788 A1 discloses an apparatus for determining a vertical position of at least one interface between a first component and at least one second component, the components being comprised as different layers in a sample container or sample tube. The disclosed apparatus comprises a first and a second sensing unit at different vertical positions, sensing light emitted by corresponding infrared ray radiation elements.

WO 2011/019576 A1 discloses the use of a single laser diode in combination with a corresponding single collimation optics for ascertaining interferents and physical dimensions in liquid samples.

US 2012/0013889 A1 discloses an apparatus for determining a vertical position of at least one interface between a first component and at least one second component, the components being comprised as different layers in a sample container or sample tube. The disclosed apparatus comprises a first and a second sensing unit at identical vertical positions.

Based on the apparatus disclosed in US 2012/0013889 A1, it is an aspect of the disclosed invention to improve the performance in interface position detection, especially where labels are attached to the sample container. Improved interface detection is provided by a laboratory automation system according to claim 1.

The laboratory automation system is adapted to process components comprised in a sample container.

The system includes at least one laboratory station functionally coupled to the apparatus. The system may include different laboratory stations, such as pre analytical stations, analytical stations and post analytical stations.

The system includes an apparatus being adapted to detect a position of an interface between a first component and a second component, the components being comprised as different layers in a conventional transparent sample container or sample tube as used in automated laboratory instrumentation. For example, the apparatus can detect horizontal interfaces between different layers within a centrifuged sample tube containing blood, such as interfaces between a serum or plasma layer and either a separation medium layer (in so called tubes) or a cruor (blood cell) layer.

The disclosed apparatus comprises a first sensing unit that includes a first laser diode that emits light having a first wavelength, the first wavelength being substantially transmitted by the sample container and a first component.

The first sensing unit further includes a first collimating optics adapted to collimate the light of the first wavelength that is generated by the first laser diode, such that the light is emitted in form of a beam having a defined diameter and direction in space. The beam may propagate substantially perpendicular to a vertical axis of the sample container, for example at an angle relative to a vertical axis of the sample container of between 85 degrees and 95 degrees, such as between 89 degrees and 91 degrees. Further, the beam may propagate substantially through the vertical axis of the sample container.

The first sensing unit further includes a first light detector, e.g. a photodiode or phototransistor, that generates a first sensing signal, for example a sensing voltage or sensing current, in response to or indicative of an intensity of light having the first wavelength that reaches the first light detector.

The apparatus further includes a second sensing unit vertically spaced by a vertical distance from the first sensing unit. The vertical distance can be specified by design and generally does not change during operation of the apparatus.

The second sensing unit includes a second laser diode that emits light having a second wavelength, the second wavelength being substantially transmitted by a sample container but blocked or absorbed by the first component.

The second sensing unit further includes a second collimating optics adapted to collimate the light of the second wavelength generated by the second laser diode, such that the light is emitted in form of a beam having a defined diameter and direction in space. The beam may propagate substantially perpendicular to a vertical axis of the sample container, for example at an angle relative to the vertical axis of the sample container of between 85 degrees and 95 degrees, such as between 89 degrees and 91 degrees. Further, the beam may propagate substantially through the vertical axis of the sample container. The resulting beam having the second wavelength and the resulting beam having the first wavelength can propagate in a parallel but vertically spaced path separated by a vertical distance between the first and the second sensing unit.

In particular embodiments, the beam generated by the first laser diode and the first collimating optics at the first wavelength and the beam generated by the second laser diode and the second collimating optics at the second wavelength have substantially identical diameters and substantially parallel propagation directions in space. Beams having substantially identical diameters and directions in space function to increase the accuracy of interface detection according to the disclosure.

The second sensing unit further comprises a second light detector generating a second sensing signal (such as voltage or current) in response to or indicative of an intensity of light having the second wavelength that reaches the second light detector.

The apparatus further comprises a driving unit, e.g. having a gripper to grip the sample container, adapted to move the first sensing unit and the second sensing unit relative to the sample container, either together or separately. In some embodiments, the driving unit provides relative motion between the first and second sensing units and the sample container in both a substantially vertical direction aligned with a central axis of a (cylindrical) sample container or tube and in a rotational direction about the central axis of the (cylindrical) sample container or tube.

The apparatus further comprises a position sensing unit, adapted to output a position sensing signal indicative of a vertical position of the sample container, e.g. relative to first sensing unit, or the second sensing unit, or any other given or know relative position. The relative position can, for example, be defined by a light barrier defining a reference vertical position.

The apparatus further comprises a vertical position determining unit that is adapted to match the first sensing signal and the second sensing signal such that first sensing signal and the second sensing signal correspond to substantially identical vertical positions. The matching reflects that the first sensing unit and the second sensing unit are vertically spaced by the given vertical distance, such that the first sensor signal and the second sensor signal at a given measurement time correspond to different vertical positions of the sample container. The matching may be done by a transformation of vertical position coordinates for one of the sensing signals.

The vertical position determining unit is further adapted to calculate or determine the vertical position of the at least one interface in response to the matched sensing signals and the position sensing signal.

Laser diode light sources make it possible to detect component interfaces even when labels are attached to the sample container. It is common practice in laboratories where the disclosed device is to be employed that medical and laboratory personnel will add multiple layers of labels (such as 2, 3, 4 or even 5 or more layers) as a sample is processed to yield an analysis result. However, even with the increased intensity provided by laser diode light sources, the detection limit of the disclosed sensing units can be reached or a less than desirable signal to noise ratio can be observed as the number of labels attached to a sample tube increases. Therefore, in particular embodiments, the disclosed apparatus further includes a mechanism to rotate the relative position of the first and second sensing units about a vertical axis of a sample tube (or at least substantially about its vertical axis) in order to reduce the number of label layers that the light emitted by the laser diodes must pass through before impinging on the light sensors. For example, the sample tube can be rotated or the first and second sensing units can be rotated around the sample tube, or both.

Wavelength specific collimation optics optimize the detection performance of the disclosed apparatus compared to devices having shared collimation optics, (i.e. beams of multiple wavelengths share an identical measurement path). Shared collimation optics are typically optimized for one of the wavelengths, and thus have reduced performance for other wavelengths. Alternatively, a compromise is made such that the collimation optics are not optimal for any of the wavelengths. Finally, by using the matching operation the measurement paths which differ due to the vertical spacing of the sensing units are virtually matched or aligned with one another, enabling the conventional interface detection e.g. on a ratio basis between light beams having different wavelengths, as disclosed in US 2012/0013889 A1.

The vertical position determining unit may further be adapted to calculate the vertical position of the at least one interface using a ratio between the matched sensing signals. After matching, a quotient between the second sensing signal and the first sensing signal (or vice versa) may be evaluated, wherein the quotient may be compared with a given threshold value. A vertical position for which the result of the comparison changes may be determined as a vertical interface position.

The components may be selected from a group consisting of air, (blood) serum or plasma, separation gel, and cruor (blood cells).

The first wavelength may range between 400 nm and 1200 nm and the second wavelength may range between 1300 nm and 1700 nm.

As mentioned above, the driving unit may be adapted to rotate the sample container around a vertical axis of the sample container. As such, the apparatus may be adapted to repeat the detecting of the vertical position of the at least one interface for the rotated sample container. This eases the interface detection in case of labels being attached to the sample container, since by means of rotating a measurement path having less label layers may be found, enhancing the signal to noise ratio.

The first collimating optics may be specifically adapted to the first wavelength and the second collimating optics may be specifically adapted to the second wavelength, e.g. by wave length specific geometric dimensioning, wave length specific materials, etc.

The driving unit is adapted to insert the sample container into a sample container carrier, into a sample container conveyor, into a sample aliquoter, into an analytical instrument, etc., wherein the process of interface detection is simultaneously performed. By performing two tasks, namely interface detection and insertion, in parallel, the overall processing time may be reduced significantly.

The apparatus and the laboratory station(s) may be functionally coupled be means of a data bus enabling data exchange between the apparatus and the laboratory station(s).

The laboratory station is adapted to operate in response to the detected vertical position of at least one interface.

The laboratory stations may be an aliquoter unit having a pipetting unit, the pipetting unit having a tip, wherein during aliquoting the aliquoter unit is adapted to control a vertical position of the tip in response to the detected vertical position of at least one interface, such that only a desired component is transmitted into secondary tubes.

The system includes a sample container transport unit adapted to transport sample containers between different laboratory stations. The sample container transport unit comprises a number, e.g. 10 to 200, of sample container carriers. The driving unit is adapted to insert a sample container into a sample container carrier parallel to detecting the vertical position of the at least one interface, thus increasing the overall processing performance.

The sample container transport unit may include a conveyor (belt), wherein the sample container carriers are attached to the conveyor.

The invention will now be described with respect to the attached drawings, wherein:
- Fig. 1: schematically depicts an apparatus for determining a vertical position of at least one interface between a first component and at least one second component being comprised in a sample container,
- Fig. 2: schematically illustrates light beams generated using collimating optics comprised in the apparatus depicted in Fig. 1,
- Fig. 3: schematically illustrates a first and a second sensing signal depending on a vertical position before matching the sensing signals,
- Fig. 4: schematically illustrates the first and the second sensing signal after matching the sensing signals,
- Fig. 5: schematically illustrates a laboratory automation system comprising the apparatus depicted in Fig. 1, and
- Fig. 6: schematically illustrates aspects of the laboratory automation system depicted in Fig. 5 in more detail.

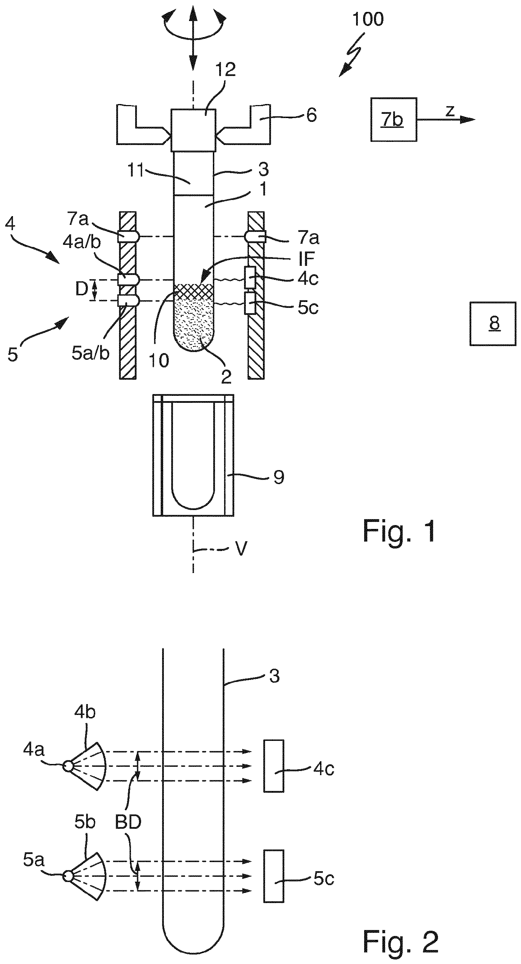

Fig. 1 schematically depicts an apparatus for determining a vertical position of an interface (IF) between a first component in form of (blood) serum 1 and a second component in form of a separating medium 10, e.g. in form of a gel. The components 1 and 10 are comprised as different layers in a sample container or sample tube 3, wherein the sample container 3 further comprises a third component in form of cruor (blood cells) 2 at the bottom and a fourth component in form of air 11 at the top.

The sample tube 3 is closed by means of a removable cap 12.

The apparatus comprises a first sensing unit 4 comprising a first laser diode 4a emitting light having a first wavelength of 800 nm. Light having this wavelength is substantially transmitted by the material of the sample container 3 and the serum 1. A corresponding first collimating optics 4b collimates the light having the first wavelength such that a vertical light beam having a diameter of approximately 0,8 mm is generated, such that the light beam propagates through the sample tube 3 and the respective component along a vertical measurement path.

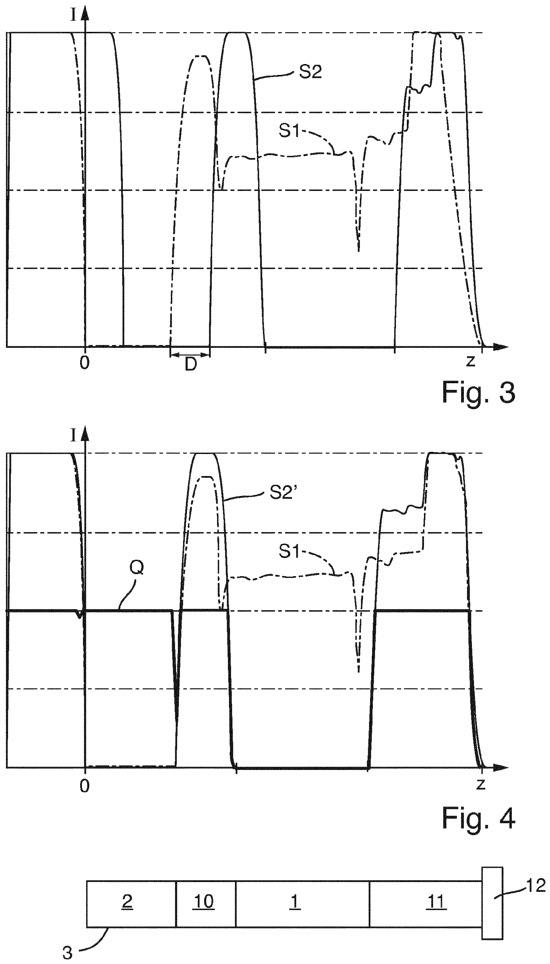

A first light detector in form of a photo diode 4c is arranged at a vertical level which is the same as the vertical level of the first laser diode 4a. The photo diode 4c generates a first sensing signal S1 (see figures 2 and 3) in response to an intensity of light having the first wavelength being applied to the photo diode 4c.

The apparatus comprises a second sensing unit 5 vertically spaced by a given vertical distance D, e.g. approximately 10 mm, from the first sensing unit 4. The second sensing unit 5 comprises a second laser diode 5a emitting light having a second wavelength of 1550 nm. Light having this wavelength is substantially transmitted by the material of the sample container 3 but blocked or absorbed by the serum 1. A corresponding second collimating optics 5b collimates the light having the second wavelength such that a vertical light beam having a diameter of approximately 0,8 mm is generated propagating through the sample tube 3 and the respective component along a vertical measurement path.

A second light detector in form of a photo diode 5c is arranged at a vertical level which is the same as the vertical level of the second laser diode 5a. The photo diode 5c generates a second sensing signal S2 (see figures 2 and 3) in response to an intensity of light having the second wavelength being applied to the photo diode 5c.

Fig. 2 schematically illustrates light beams generated using the collimating optics 4b and 5b, respectively.

As depicted, as a result of the collimating optics 5a and 5b the beam generated by the first laser diode 4a at the first wavelength and the beam generated by the second laser diode 5a at the second wavelength have substantially identical diameters BD and substantially parallel propagation directions in space. Beams having substantially identical diameters and directions in space function to increase the accuracy of interface detection compared with embodiments having no collimation optics.

The apparatus further comprises a driving unit in form of a pick-and-place unit 6 for vertically moving the sample container 3 relative to the first and second sensing unit 4 and 5. The pick-and-place unit 6 is further adapted to rotate the sample container 3 around a vertical axis V of the sample container 3.

The apparatus further comprises a position sensing unit in form of a light barrier 7a and a path sensor 7b. The path sensor 7b is functionally coupled to the pick-and-place unit 6 and measures a vertical distance of a movement caused by the pick-and-place unit 6. If the pick-and-place unit 6 vertically moves the sample container 3 from a vertical level above the light barrier 7a towards the light barrier 7a, the light barrier 7a detects when the sample container 3 disrupts the light path of the light barrier 7a. This vertical position may be defined as a zero or reference position, i.e. a position sensing signal z output from the position sensing unit (here path sensor 7b) for this reference position has a defined reference value, e.g. zero. Thus, the position sensing unit outputs a position sensing signal z indicative of a vertical position of the sample container 3, wherein the vertical position of the light barrier 7a is defined as a vertical reference position.

A vertical position determining unit 8, e.g. in form of a microprocessor, is functionally coupled to the first and the second sensing unit 4 and 5, the pick-and-place unit 6 and the position sensing unit 7a and 7b.

The vertical position determining unit 8 controls the pick-and-place unit 6 such that the sample container 3 is sampled along a vertical measurement path. The resulting first sensing signal S1 and the resulting second sensing signal S2 in intensity units as a function of the position sensing signal z are depicted in fig. 3. To simplify the diagram, the value z = 0 of the position sensing signal z has been chosen such that it corresponds to the bottom end of the sample container 3.

As depicted, the first and second sensing signals S1 and S2 are horizontally misaligned by z = D due to the vertical distance D between the sensing units 4 and 5.

Light having the first wavelength and light having the second wavelength is respectively blocked or absorbed by the cruor 2 and respectively transmitted by the separating medium 10 and air 11. Only the serum 2 has transmission characteristics depending of the chosen wavelength. Light having the first wavelength is transmitted by the serum 1 but light having the second wavelength is blocked or absorbed by the serum 1.

Before analyzing the sensing signals S1 and S2, the vertical position determining unit 8 matches the first sensing signal S1 and the second sensing signal S2. To achieve this, the vertical position determining unit 8 horizontally shifts the sensing signal S2 to the left by z = D, resulting in the matched sensing signal S2', see fig. 4.

After matching the sensing signals S1 and S2, the vertical position determining unit 8 computes a quotient Q (including signal smoothing, limiting, etc.) between the matched second sensing signal S2' and the first sensing signal S1, wherein the quotient Q is compared with a given threshold value. A vertical position for which the result of the comparison changes is determined as a vertical interface position. As such, the vertical positions of the interfaces between the components (11, 1) and (1, 10) are computable. For further details regarding this aspect reference is made to US 2012/0013889 A1.

The computed vertical interface positions may be used in further processing, e.g. when pipetting the sample container 3.

If labels are glued to the sample container 3, the sensing signals S1 and S2 may not have sufficient signal strength. In this case, the driving unit 6 may rotate the sample container 3 around the vertical axis V of the sample container 3 to cause a measurement path eventually crossing a decreased number of label layers and may repeat the measurement. As such, a measurement path having less label layers may be found, thus increasing the signal-to-noise ratio if the sensing signals.

The driving unit 6 is further adapted to insert the sample container 3 into a conventional sample container carrier 9. By performing two tasks, namely interface detection and carrier insertion, in parallel, the overall processing time may be reduced.

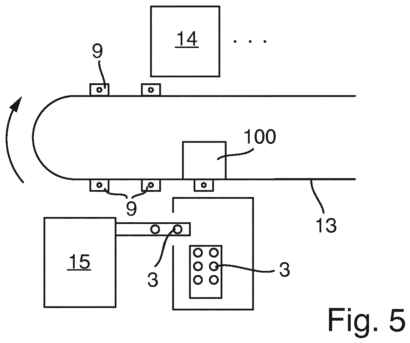

Fig. 5 schematically illustrates a laboratory automation system comprising the apparatus 100, a centrifuge 15, and an exemplary laboratory station in form of an aliquoter unit 14. The apparatus 100 and the aliquoter unit 14 are functionally coupled by means of a conventional data or field bus. Self-evidently, the system may include further laboratory stations, such as pre analytical stations, analytical stations and post analytical stations.

The sample containers 3 are supplied after being centrifuged by means of the centrifuge 15 or already centrifuged within racks.

The aliquoter unit 14 transfers part of the serum 1 to one or more secondary tubes (not shown). The aliquoter unit 14 conventionally includes a pipetting unit (not shown), the pipetting unit having a tip (not shown), wherein during aliquoting the aliquoter unit 14 is adapted to control a vertical position of the tip in response to the detected vertical position of the interface IF, such that the tip remains within the serum 1 above the separating medium 10.

The system further includes a sample container transport unit adapted to transport sample containers 3 between the apparatus 100, the aliquoter unit 14 and further laboratory stations (not shown). The sample container transport unit includes a number of sample container carriers 9 and a conveyor 13, wherein the sample container carriers 9 are attached to the conveyor 13.

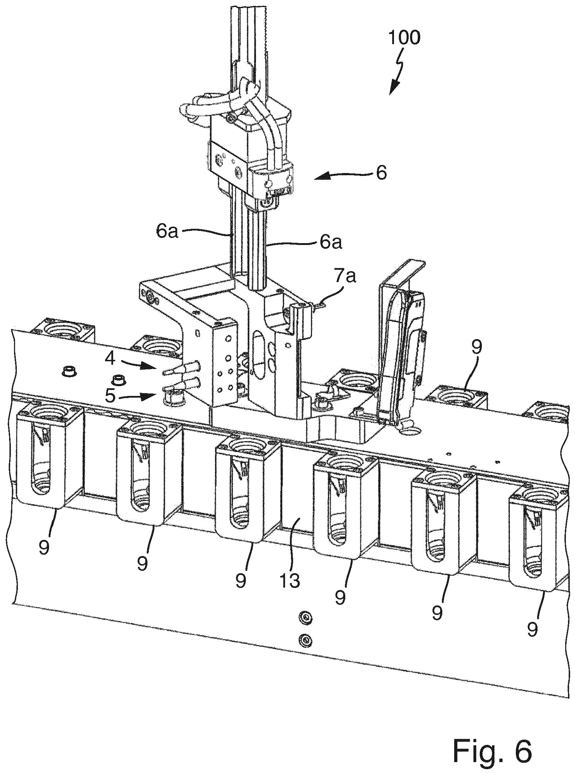

Fig. 6 schematically illustrates the driving unit or pick-and-place unit 6, the first and second sensing unit 4 and 5, the light barrier 7a and the sample container transport unit in more detail.

The driving unit or pick-and-place unit 6 includes a gripper 6a to grip the sample container 3. The driving unit or pick-and-place unit 6 further includes means to provide a relative motion between the first and second sensing units 4 and 5 and the sample container 3 in both a substantially vertical direction aligned with the central axis V of the cylindrical sample container 3 and in a rotational direction about the central axis V of the sample container 3.

The driving unit or pick-and-place unit 6 inserts a sample container 3 into a corresponding sample container carrier 9, wherein the apparatus 100 simultaneously detects the vertical position of the interface IF. During insertion the conveyor 13 is stopped. After insertion the conveyor 13 is moved such that an empty sample container carrier 9 is placed under the pick-and-place unit 6, such that a further sample container 3 may be inserted into the empty sample container carrier 9.

The embodiments improve the performance in position detection, especially in view of labels attached to the sample container 3, since laser diodes having wave length specific optics can be used. The necessary vertical displacement of the measurement paths is compensated by virtually matching the different measurement paths.

The embodiments further improve the overall processing performance, since interface detection and insertion of sample in corresponding sample container carriers is done simultaneously.

## Claims

1. Laboratory automation system for processing components comprised in a sample container (3), comprising
- an apparatus (100) for determining a vertical position of at least one interface (IF) between a first component (1) and at least one second component (10), the components (1, 10) being comprised as different layers in a sample container (3), the apparatus (100) comprising:
- a first sensing unit (4) comprising:
- a first laser diode (4a) adapted to emit light having a first wavelength, which is substantially transmitted by the sample container (3) and the first component (1),
- a first collimating optics (4b) adapted to collimate the light having the first wavelength, such that the light is emitted in form of a beam having a defined diameter and direction in space, and
- a first light detector (4c) adapted to generate a first sensing signal (S1) in response to an intensity of light having the first wavelength being applied to the first light detector (4c),
- a second sensing unit (5) vertically spaced by a given vertical distance (D) from the first sensing unit (4) and comprising:
- a second laser diode (5a) adapted to emit light having a second wavelength, which is substantially transmitted by the sample container (3) but blocked by the first component (1),
- a second collimating optics (5b) adapted to collimate the light having the second wavelength, such that the light is emitted in form of a beam having a defined diameter and direction in space, wherein the resulting beam having the second wavelength and the resulting beam having the first wavelength propagate in parallel but vertically spaced paths separated by the given vertical distance (D), and
- a second light detector (5c) adapted to generate a second sensing signal (S2) in response to an intensity of light having the second wavelength being applied to the second light detector (5c),
- a driving unit (6) adapted to move the sample container (3) relative to the first sensing unit (4) and the second sensing unit (5),
- a position sensing unit (7a, 7b), adapted to output a position sensing signal (z) indicative of a vertical position of the sample container (3), and
- a vertical position determining unit (8), adapted to
- match the first sensing signal (S1) and the second sensing signal (S2) such that first sensing signal (S1) and the second sensing signal (S2) correspond to identical vertical positions, and
- determine the vertical position of the at least one interface (IF) in response to the matched sensing signals (S1, S2') and the position sensing signal (z),
- wherein the laboratory automation system further comprises at least one laboratory station (14) functionally coupled to the apparatus (100), wherein the at least one laboratory station (14) is adapted to operate in response to the detected vertical position of the at least one interface, and
- wherein the laboratory automation system further comprises a sample container transport unit adapted to transport the sample container (3) between different laboratory stations, wherein the sample container transport unit comprises a number of sample container carriers (9), wherein the driving unit (6) is adapted to insert a sample container (3) into a sample container carrier (9) parallel to detecting the vertical position of the at least one interface (IF).

2. Laboratory automation system according to claim 1, **characterized in that**
- the vertical position determining unit (8) is adapted to calculate the vertical position of the at least one interface (IF) using a ratio (Q) between the matched sensing signals (S1, S2').

3. Laboratory automation system according to claim 1 or 2, **characterized in that**
- the first and the second component (1, 10) are selected from a group consisting of air, serum and separation gel.

4. Laboratory automation system according to anyone of the preceding claims, **characterized in that**
- the first wavelength ranges between 400 nm and 1200 nm and the second wavelength ranges between 1300 nm and 1700 nm.

5. Laboratory automation system according to anyone of the preceding claims, **characterized in that**
- the driving unit (6) is adapted to rotate the sample container (3) around a vertical axis (V) of the sample container (3), wherein the apparatus is adapted to repeat the detecting of the vertical position of the at least one interface (IF) for the rotated sample container (3).

6. Laboratory automation system according to anyone of the preceding claims, **characterized in that**
- the first collimating optics (4b) is specifically adapted to the first wavelength and the second collimating optics (5b) is specifically adapted to the second wavelength.

7. Laboratory automation system according to anyone of the preceding claims, **characterized by**
- a light barrier (7a) adapted to detect the introduction of a sample container (3) into the apparatus, wherein the apparatus is adapted to activate the first and the second sensing unit (4, 5) when the introduction is detected.

8. Laboratory automation system according to anyone of the preceding claims, **characterized in that**
- at least one of the laboratory stations is an aliquoter unit (14) having a pipetting unit, the pipetting unit having a tip, wherein during aliquoting the aliquoter unit (14) is adapted to control a vertical position of the tip in response to the detected vertical position of the at least one interface (IF).

9. Laboratory automation system according to anyone of the preceding claims, **characterized in that**
- the sample container transport unit comprises a conveyor (13), the sample container carriers (9) being attached to the conveyor (13).

## Patentansprüche

1. Laborautomatisierungssystem zur Bearbeitung von Komponenten, die in einem Probenbehälter (3) vorliegen, aufweisend
- eine Einrichtung (100) zum Bestimmen einer vertikalen Position mindestens einer Grenzfläche (IF) zwischen einer ersten Komponente (1) und mindestens einer zweiten Komponente (10), wobei die Komponenten (1, 10) als unterschiedliche Schichten in einem Probenbehälter (3) vorliegen, wobei die Einrichtung (100) aufweist:
- eine erste Messeinheit (4), aufweisend:
- eine erste Laserdiode (4a), die zum Emittieren von Licht mit einer ersten Wellenlänge ausgebildet ist, das im Wesentlichen durch den Probenbehälter (3) und die erste Komponente (1) durchgelassen wird,
- eine erste Kollimationsoptik (4b), die zur Kollimation des Lichts mit der ersten Wellenlänge ausgebildet ist, so dass das Licht in Form eines Strahls mit einem definierten Durchmesser und einer definierten Richtung im Raum emittiert wird, und
- einen ersten Lichtdetektor (4c), der zum Erzeugen eines ersten Messsignals (S1) in Abhängigkeit von einer Intensität von Licht mit der ersten Wellenlänge, das auf den ersten Lichtdetektor (4c) trifft, ausgebildet ist,
- eine zweite Messeinheit (5), die um einen vorgegebenen vertikalen Abstand (D) von der ersten Messeinheit (4) vertikale beabstandet ist und aufweist:
- eine zweite Laserdiode (5a), die zum Emittieren von Licht mit einer zweiten Wellenlänge ausgebildet ist, das im Wesentlichen durch den Probenbehälter (3) durchgelassen, aber von der ersten Komponente (1) blockiert wird,
- eine zweite Kollimationsoptik (5b), die zur Kollimation des Lichts mit der zweiten Wellenlänge ausgebildet ist, so dass das Licht in Form eines Strahls mit einem definierten Durchmesser und einer definierten Richtung im Raum emittiert wird, wobei der resultierende Strahl mit der zweiten Wellenlänge und der resultierende Strahl mit der ersten Wellenlänge sich parallel zueinander, aber auf um den vorgegebenen vertikalen Abstand (D) getrennten vertikal beabstandeten Wegen ausbreiten, und
- einen zweiten Lichtdetektor (5c), der zum Erzeugen eines zweiten Messsignals (S2) in Abhängigkeit von einer Intensität von Licht mit der zweiten Wellenlänge, das auf den zweiten Lichtdetektor (5c) trifft, ausgebildet ist,
- eine Antriebseinheit (6), die zum Versetzen des Probenbehälter (3) relativ zur ersten Messeinheit (4) und zur zweiten Messeinheit (5) ausgebildet ist,
- eine Positionsmesseinheit (7a, 7b), die zum Ausgeben eines Positionsmesssignals (z) ausgebildet ist, das eine vertikale Position des Probenbehälters (3) angibt, und
- eine Bestimmungseinheit (8) für eine vertikale Position, die ausgebildet ist zum
- Abstimmen des ersten Messsignals (S1) und des zweiten Messsignals (S2), so dass das erste Messsignal (S1) und das zweite Messsignal (S2) identischen vertikalen Positionen entsprechen, und
- Bestimmen der vertikalen Position der mindestens einen Grenzfläche (IF) in Abhängigkeit von den abgestimmten Messsignalen (S1, S2') und dem Positionsmesssignal (z),
- wobei das Laborautomatisierungssystem weiter mindestens eine Laborstation (14) umfasst, die funktionell mit der Einrichtung (100) gekoppelt ist, wobei die mindestens eine Laborstation (14) zum Betrieb in Abhängigkeit von der detektierten vertikalen Position der mindestens einen Grenzfläche ausgebildet ist, und
- wobei das Laborautomatisierungssystem weiter eine Probenbehältertransporteinheit umfasst, die zum Transportieren des Probenbehälters (3) zwischen verschiedenen Laborstationen ausgebildet ist, wobei die Probenbehältertransporteinheit eine Anzahl an Probenbehälterträgern (9) umfasst, wobei die Antriebseinheit (6) zum Einsetzen eines Probenbehälters (3) in einen Probenbehälterträger (9) parallel zum Detektieren der vertikalen Position der mindestens einen Grenzfläche (IF) ausgebildet ist.

2. Laborautomatisierungssystem nach Anspruch 1, **dadurch gekennzeichnet, dass**
- die Bestimmungseinheit (8) für eine vertikale Position zum Berechnen der vertikalen Position der mindestens einen Grenzfläche (IF) unter Verwendung eines Verhältnisses (Q) zwischen den abgestimmten Messsignalen (S1, S2') ausgebildet ist.

3. Laborautomatisierungssystem nach Anspruch 1 oder 2, **dadurch gekennzeichnet, dass**
- die erste und die zweite Komponente (1, 10) ausgewählt sind aus einer Gruppe bestehend aus Luft, Serum und Trenngel.

4. Laborautomatisierungssystem nach einem der vorhergehenden Ansprüche, **dadurch gekennzeichnet, dass**
- die erste Wellenlänge im Bereich zwischen 400 nm und 1200 nm und die zweite Wellenlänge im Bereich zwischen 1300 nm und 1700 nm liegt.

5. Laborautomatisierungssystem nach einem der vorhergehenden Ansprüche, **dadurch gekennzeichnet, dass**
- die Antriebseinheit (6) zum Drehen des Probenbehälters (3) um eine vertikale Achse (V) des Probenbehälters (3) ausgebildet ist, wobei die Einrichtung zum Wiederholen der Detektion der vertikalen Position der mindestens einen Grenzfläche (IF) bei gedrehten Probenbehälter (3) ausgebildet ist.

6. Laborautomatisierungssystem nach einem der vorhergehenden Ansprüche, **dadurch gekennzeichnet, dass**
- die erste Kollimationsoptik (4b) spezifisch für die erste Wellenlänge ausgebildet ist und die zweite Kollimationsoptik (5b) spezifisch für die zweite Wellenlänge ausgebildet ist.

7. Laborautomatisierungssystem nach einem der vorhergehenden Ansprüche, **gekennzeichnet durch**
- eine Lichtschranke (7a), die zum Detektieren der Einführung eines Probenbehälters (3) in die Einrichtung ausgebildet ist, wobei die Einrichtung zum Aktivieren der ersten und der zweiten Messeinheit (4, 5) ausgebildet ist, wenn die Einführung detektiert wurde.

8. Laborautomatisierungssystem nach einem der vorhergehenden Ansprüche, **dadurch gekennzeichnet, dass**
- mindestens eine der Laborstationen als Aliquotierungseinheit (14) mit einer Pipettierungseinheit ausgebildet ist und die Pipettierungseinheit eine Spitze aufweist, wobei beim Aliquotieren die Aliquotierungseinheit (14) zum Ansteuern einer vertikalen Position der Spitze in Abhängigkeit von der detektierten vertikalen Position der mindestens einen Grenzfläche (IF) ausgebildet ist.

9. Laborautomatisierungssystem nach einem der vorhergehenden Ansprüche, **dadurch gekennzeichnet, dass**
- die Probenbehältertransporteinheit eine Fördereinrichtung (13) umfasst, wobei die Probenbehälterträger (9) an der Fördereinrichtung (13) angebracht sind.

## Revendications

1. Système d'automatisation de laboratoire pour traiter des composants compris dans un contenant d'échantillon (3), comprenant
- un appareil (100) pour déterminer une position verticale d'au moins une interface (IF) entre un premier composant (1) et au moins un second composant (10), les composants (1, 10) étant compris en tant que couches différentes dans un contenant d'échantillon (3), l'appareil (100) comprenant :
- une première unité de détection (4) comprenant :
- une première diode laser (4a) adaptée pour émettre une lumière ayant une première longueur d'onde, qui est sensiblement transmise par le contenant d'échantillon (3) et le premier composant (1),
- une première optique de collimation (4b) adaptée pour collimater la lumière ayant la première longueur d'onde, de sorte que la lumière soit émise sous forme d'un faisceau ayant un diamètre et une direction définis dans l'espace, et
- un premier détecteur de lumière (4c) adapté pour générer un premier signal de détection (S1) en réponse à une intensité de lumière ayant la première longueur d'onde appliquée au premier détecteur de lumière (4c),
- une seconde unité de détection (5) espacée verticalement d'une distance verticale donnée (D) de la première unité de détection (4) et comprenant :
- une seconde diode laser (5a) adaptée pour émettre une lumière ayant une seconde longueur d'onde, qui est transmise sensiblement par le contenant d'échantillon (3) mais bloquée par le premier composant (1),
- une seconde optique de collimation (5b) adaptée pour collimater la lumière ayant la seconde longueur d'onde, de sorte que la lumière soit émise sous forme d'un faisceau ayant un diamètre et une direction définis dans l'espace, dans lequel le faisceau résultant ayant la seconde longueur d'onde et le faisceau résultant ayant la première longueur d'onde se propagent dans des chemins parallèles mais espacés verticalement de la distance verticale donnée (D), et
- un second détecteur de lumière (5c) adapté pour générer un second signal de détection (S2) en réponse à une intensité de lumière ayant la seconde longueur d'onde appliquée au second détecteur de lumière (5c),
- une unité d'entraînement (6) adaptée pour déplacer le contenant d'échantillon (3) par rapport à la première unité de détection (4) et la seconde unité de détection (5),
- une unité de détection de position (7a, 7b), adaptée pour fournir en sortie un signal de détection de position (z) indicatif d'une position verticale du contenant d'échantillon (3), et
- une unité de détermination de position verticale (8), adaptée pour
- faire concorder le premier signal de détection (S1) et le second signal de détection (S2) de sorte que le premier signal de détection (S1) et le second signal de détection (S2) correspondent à des positions verticales identiques, et
- déterminer la position verticale de l'au moins une interface (IF) en réponse aux signaux de détection concordés (S1, S2') et au signal de détection de position (z),
- dans lequel le système d'automatisation de laboratoire comprend en outre au moins une station de laboratoire (14) couplée fonctionnellement à l'appareil (100), dans lequel l'au moins une station de laboratoire (14) est adaptée pour fonctionner en réponse à la position verticale détectée de l'au moins une interface, et
- dans lequel le système d'automatisation de laboratoire comprend en outre une unité de transport de contenant d'échantillon adaptée pour transporter le contenant d'échantillon (3) entre des stations de laboratoire différentes, dans lequel l'unité de transport de contenant d'échantillon comprend un certain nombre de porte-contenants d'échantillon (9), dans lequel l'unité d'entraînement (6) est adaptée pour insérer un contenant d'échantillon (3) dans un porte-contenant d'échantillon (9) en parallèle à la détection de la position verticale de l'au moins une interface (IF).

2. Système d'automatisation de laboratoire selon la revendication 1, **caractérisé en ce que**
- l'unité de détermination de position verticale (8) est adaptée pour calculer la position verticale de l'au moins une interface (IF) à l'aide d'un rapport (Q) entre les signaux de détection concordés (S1, S2').

3. Système d'automatisation de laboratoire selon la revendication 1 ou 2, **caractérisé en ce que**
- les premier et second composants (1, 10) sont choisis dans un groupe constitué de l'air, d'un sérum et d'un gel de séparation.

4. Système d'automatisation de laboratoire selon l'une quelconque des revendications précédentes, **caractérisé en ce que**
- la première longueur d'onde va de 400 nm à 1 200 nm et la seconde longueur d'onde va de 1 300 nm à 1 700 nm.

5. Système d'automatisation de laboratoire selon l'une quelconque des revendications précédentes, **caractérisé en ce que**
- l'unité d'entraînement (6) est adaptée pour mettre en rotation le contenant d'échantillon (3) autour d'un axe vertical (V) du contenant d'échantillon (3), dans lequel l'appareil est adapté pour répéter la détection de la position verticale de l'au moins une interface (IF) pour le contenant d'échantillon (3) mis en rotation.

6. Système d'automatisation de laboratoire selon l'une quelconque des revendications précédentes, **caractérisé en ce que**
- la première optique de collimation (4b) est adaptée spécifiquement à la première longueur d'onde et la seconde optique de collimation (5b) est adaptée spécifiquement à la seconde longueur d'onde.

7. Système d'automatisation de laboratoire selon l'une quelconque des revendications précédentes, **caractérisé par**
- une barrière photoélectrique (7a) adaptée pour détecter l'introduction d'un contenant d'échantillon (3) dans l'appareil, dans lequel l'appareil est adapté pour activer la première et la seconde unité de détection (4, 5) lorsque l'introduction est détectée.

8. Système d'automatisation de laboratoire selon l'une quelconque des revendications précédentes, **caractérisé en ce que**
- au moins l'une des stations de laboratoire est une unité d'aliquoteur (14) ayant une unité de pipetage, l'unité de pipetage ayant une pointe, dans lequel pendant l'aliquotage, l'unité d'aliquoteur (14) est adaptée pour commander une position verticale de la pointe en réponse à la position verticale détectée de l'au moins une interface (IF).

9. Système d'automatisation de laboratoire selon l'une quelconque des revendications précédentes, **caractérisé en ce que**
- l'unité de transport de contenant d'échantillon comprend un convoyeur (13), les porte-contenants d'échantillon (9) étant fixés au convoyeur (13).
